# EUROPÄISCHE PATENTANMELDUNG

(11) **EP 0 230 268 A2**
(43) Veröffentlichungstag der Anmeldung: **29.07.1987**
(21) Anmeldenummer: 87100438.8
(22) Anmeldetag: 15.01.1987
(51) Int. Cl.: C07D 249/08, C07D 233/60, C07D 233/61, C07D 405/06, A01N 43/50, A01N 43/653

(54) **Halogenierte Azolverbindungen und diese enthaltende Fungizide**

(30) Priorität: 20.01.1986 DE 3601430
(71) Anmelder: BASF Aktiengesellschaft, 67063 Ludwigshafen (DE)
(72) Erfinder: Schulz, Günter, Dr., D-6700 Ludwigshafen (DE); Rentzea, Costin, Dr., D-6900 Heidelberg (DE); Pommer, Ernst-Heinrich, Dr., D-6703 Limburgerhof (DE); Ammermann, Eberhard, Dr., D-6700 Ludwigshafen (DE); Jung, Johann, Prof.Dr., D-6703 Limburgerhof (DE); Rademacher, Wilhelm, Dr., D-6703 Limburgerhof (DE)

(57) **Zusammenfassung**

Azolverbindungen der Formel 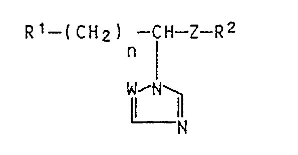 worin
R¹ für eine Gruppe der Formel 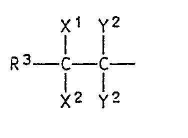 bedeutet, wobei R³, X¹, X², Y¹ und Y² Wasserstoff, Halogen oder Alkyl bedeuten,
n eine ganze Zahl von 1 bis 3 bedeutet,
W ≧CH oder ≧N ist,
R² tert.-Butyl, 5-Methyl-1,3-dioxan-5-yl, ggf. substituiertes 1-Methylcyclohexyl oder ggf. substituiertes 2-Methyltetrahydropyran-2-yl oder die Gruppe 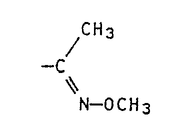 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls substituiert sein kann, und
R⁵ Wasserstoff, Alkanoyl oder Benzoyl bedeutet,
sowie deren für Pflanzen verträgliche Säureadditionssalze und Metall-Komplexe und diese Verbindungen enthaltende Fungizide und Wachstumsregulatoren.

## Beschreibung

Die Erfindung betrifft neue Azolverbindungen, Verfahren zu ihrer Herstellung und die Verwendung der Wirkstoffe zur Bekämpfung von pathogenen Pilzen und zur Regulierung des Pflanzenwachstums.

Es ist bekannt, daß 2,2-Dimethyl-4-(1H-1,2,4-triazol-1-yl)-6-hepten-3-on fungizide Eigenschaften besitzt (DE-OS 2 638 470 und DE-OS 2 805 227). Die Wirkung dieser Substanz ist jedoch bei manchen Arten und bei niedrigen Aufwandmengen nicht immer ausreichend.

Es wurden nun Verbindungen gefunden, die eine hohe fungizide und pflanzenwachstumsregulierende Wirkung bei guter Pflanzenverträglichkeit aufweisen, und zwar Verbindungen der Formel I 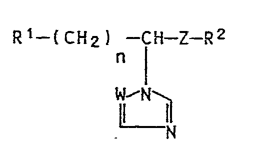 worin
R¹ für eine Gruppe der Formel 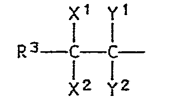 steht, wobei R³, X¹, X², Y¹ und Y² gleich oder verschieden sind und für Wasserstoff, Fluor, Chlor, Brom, Methyl oder Ethyl stehen, mit der Maßgabe, daß immer mindestens ein X und mindestens ein Y Fluor, Chlor oder Brom bedeutet,
n eine ganze Zahl von 1 bis 3 bedeutet,
W ≧CH oder ≧N ist,
R² tert.-Butyl, 5-Methyl-1,3-dioxan-5-yl, ggf. durch Methyl substituiertes 1-Methylcyclohexyl oder ggf. durch Methyl substituiertes 2-Methyltetrahydropyran-2-yl oder die Gruppe 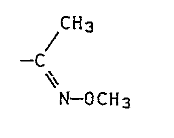 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
R⁵ Wasserstoff, C₂-C₄-Alkanoyl oder Benzoyl bedeutet,
sowie deren für Pflanzen verträgliche Säureadditionssalze und Metall-Komplexe.

Die neuen Verbindungen enthalten chirale Zentren. In den Verbindungen der Formel I sind das azolsubstituierte Kohlenstoffatom und das benachbarte durch Sauerstoff substituierte Kohlenstoffatom chiral, weiterhin sind in R¹ gegebenenfalls die halogensubstituierten Kohlenstoffe chiral. Die Wirkstoffe fallen demgemäß grundsätzlich als Enantiomerengemische an, die in die optisch aktiven Enantiomeren getrennt werden können. Im allgemeinen fallen die Wirkstoffe bedingt durch die Anwesenheit von zwei oder mehr chiralen Zentren als Diastereomerengemische an, die in üblicher Weise, z.B. durch Chromatographie oder Kristallisation in die einzelnen, einheitlichen Diastereomeren getrennt werden können. Für die Anwendung der Verbindungen als Funigzide oder als Pflanzenwachstumsregulatoren ist jedoch eine Trennung der Enantiomeren oder Diastereomeren normalerweise nicht erforderlich. Die Erfindung umfaßt sowohl die optisch aktiven einheitlichen Substanzen als auch deren Gemische.

Geeignete Säureadditionssalze sind beispielsweise die Chloride, Bromide, Sulfate, Methylsulfonate, Nitrate, Phosphate, Oxalate oder Dodecylbenzolsulfonate. Die Wirksamkeit der Salze geht auf das Kation zurück, so daß die Wahl des Anions beliebig ist.

Die Azolverbindungen der Formel I gemäß Anspruch 1, worin Z die Gruppe =CR⁴-OR⁵ bedeutet und R⁴ Wasserstoff bedeutet, werden vorzugsweise entweder durch stereoselektive Reduktion der Ketone der Formel II 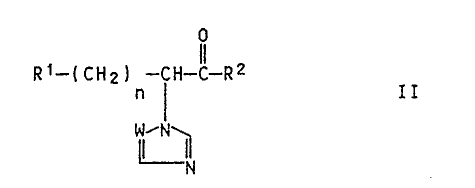 oder durch stereoselektive Reduktion der Alkene der Formel III 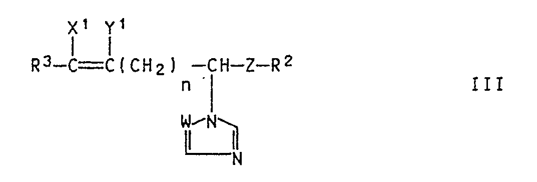 wobei Z für C=O steht und anschließende Fluorierung, Chlorierung oder Bromierung gemäß Anspruch 4 und gegebenenfalls Acylierung gemäß Anspruch 2 dargestellt.

Dabei entstehen bei der Reduktion mit komplexen Hydriden, vorzugsweise mit Natriumborhydrid in einem protischen Verdünnungsmittel, vorzugsweise Methanol, Ethanol oder i-Propanol, stark überwiegend die R*,R*-diastereomeren Alkohole der Formel I wobei in der Gruppe Z = CR⁴OR⁵, R⁴ und R⁵ Wasserstoff sind. M vorwiegend die diastereomeren Triazolylalkohole der R*,S*-Konfiguration, wenn man die Ketone der Formel II reduziert mit
a) mit sekundären Alkoholaten oder
b) mit Alkylmagnesiumhalogeniden oder
c) mit Wasserstoff in Gegenwart geeigneter Hydrierkatalysatoren.

Das gleiche gilt für die Reduktion der Alkenketone der Formel III, wenn die Gruppe Z die Bedeutung 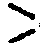 C=O hat. Zur Überführung in die Verbindungen der Formel I wird anschließend fluoriert chloriert oder bromiert.

Zur Bedeutung der Konfigurationsbezeichnungen R*R* bzw. R*S* sei folgende Erläuterung gegeben. Es werden nur die Stereozentren am azolverknüpften Kohlenstoff und am direkt benachbarten, sauerstoffverknüpften Kohlenstoff berücksichtigt. Alle anderen gegebenenfalls vorhandenen chiralen Zentren werden für die Bezeichnungsweise vernachlässigt. D.h., es wird so verfahren wie bei Verbindungen mit zwei verschiedenen chiralen Zentren, die vier Stereoisomere bilden, deren Konfiguration nach dem Cahn-Ingold-Prelog-System bezeichnet werden kann (siehe z.B. D. Seebach und V. Prelog, Angew. Chem. 94, 696 (1982) und dort angegebene Literatur). Hierbei stellen zwei Enantiomere der Konfigurationen R, R und S, S ein Diastereomer mit der Bezeichnung R*, R* dar. Das andere Diastereomer mit der Bezeichnung R*, S* setzt sich aus den beiden Enantiomeren mit den Konfigurationen R, S und S, R zusammen.

Die resultierenden Alkohole der Formel I mit Z = CHOH enthalten einen erheblich höheren Anteil der Diastereomeren mit R*,S*-Konfiguration als derjenigen mit R*,R*-Konfiguration. Der Anteil der R*,R*-Diastereomeren im Gemisch liegt in der Regel unter 30%. Reine R*,S*-Diastereomeren können daraus durch einfaches Waschen der Rohprodukte mit geeigneten Lösungsmitteln, wie z.B. Diisopropylether, oder durch Umkristallisieren oder durch andere, übliche Reinigungsschritte, z.B. Chromatographie erhalten werden.

Zur stereoselektiven Reduktion der Ketone der Formel II zu den R*,S*-Diastereomeren der Formel I mit Z = CHOH geht man nach a) folgendermaßen vor:

Die Ketone der Formel II (1 Moläquivalent) werden mit sekundären Alkoholaten (vorzugsweise 0,3 bis 1,5 Moläquivalente), vorzugsweise des Aluminiums, wie z.B. Aluminiumisopropylat, Aluminiumbutylat-(2) oder Aluminiumcyclohexylat in Gegenwart eines Verdünnungsmittels bei 60 bis 160°C, vorzugsweise bei der Siedetemperatur des Verdünnungsmittels umgesetzt. Als Verdünnungsmittel eignen sich inerte organische Lösungsmittel, insbesondere Alkohole wie Isopropanol oder Cyclohexanol. Die entstandenen diastereomeren Alkoholate werden sodann mit Hilfe von Säuren in üblicher Weise zu den freien Alkoholen der Formel I mit Z = CHOH hydrolysiert.

Für die stereoselektive Reduktion gemäß b) des oben erwähnten Verfahrens werden die Ketone der Formel II (1 Moläquivalent) mit Alkylmagnesiumhalogeniden, die mindestens ein β-ständiges H-Atom enthalten, vorzugsweise mit 0,7 bis 1,5 Moläquivalent Halogenid, in Gegenwart eines Verdünnungsmittels bei Temperaturen zwischen 0 und 120°C, umgesetzt. Als Alkylmagnesiumhalogenide kommen z.B. Ethylmagnesiumchlorid, Isopropylmagnesiumbromid, n-Propylmagnesiumbromid oder Isobutylmagnesiumchlorid in Frage. Als Lösungsmittel für die Grignard-Reduktion kommen bevorzugt Ether in Frage, wie Diethylether, Di-n-propylether, Tetrahydrofuran oder Anisol, ferner tertiäre Amine wie N,N-Diethylanilin sowie Phosphorsäuretris(dimethylamid). Vorzugsweise kann man die Reaktion auch im Gemisch dieser Lösungsmittel mit aliphatischen oder aromatischen Kohlenwasserstoffen wie n-Hexan oder Toluol durchführen. Die Reaktionstemperaturen lassen sich je nach Lösungsmittel zwischen 0 und 120°C variieren, bevorzugt sind Temperaturen zwischen 30 und 100°C.

Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extraktion, Umkristallisieren oder Chromatographie gereinigt.

Für die stereoselektive Reduktion gemäß c) erfolgt die katalytische Hydrierung am besten an einem Edelmetallkontakt. Hierzu sind die Metalle der Platingruppe am besten geeignet. Man kann diese Metalle auf inerten Trägern niederschlagen, wobei ein Metallgehalt von 0,5 bis 10 % im allgemeinen ausreichend ist. Unter Umständen kann man auch die reinen Metalle, zweckmäßig in fein verteilter Form einsetzen. Als besonders geeignet hat sich ein kolloid verteiltes Rutheniumoxidhydrat erwiesen, das durch Fällung aus einer wäßrigen Rutheniumtrichloridlösung beim pH = 8 gewonnen wurde. Die oxidische Form geht unter den Reaktionsbedingungen in den aktiven Katalysator über.

Als Lösungsmittel dienen vorzugsweise Ether wie Dioxan, Tetrahydrofuran oder Glycoldimethylether.

Die Temperatur läßt sich in weiten Grenzen verändern. Als besonders geeignet erwiesen sich Temperaturen zwischen 100 und 150°C. Bei diesen Temperaturen muß man unter Druck arbeiten; es genügen Drücke von 10 bis 30 bar. Unter höheren Drücken leidet die Selektivität.

Die Kontaktmenge ist nicht kritisch und hängt neben der Aktivität von der Reaktionstemperatur und dem Wasserstoffpartialdruck ab.

Die Acylierung der Verbindungen der Formel I mit Z in der Bedeutung =CR⁴-OR⁵ und R⁴ = H und R⁵ = H erfolgt wie weiter unten für die Verbindungen der Formel I mit R⁴ = H beschrieben.

Die Azolverbindungen der Formel I gemäß Anspruch 1, mit der Maßgabe, daß R⁴ nicht Wasserstoff ist, werden durch Umsetzung der Ketone der Formel II 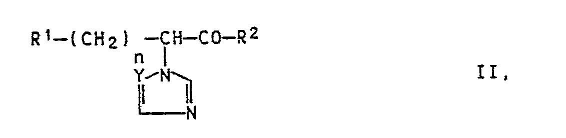 in der R¹, R², n und W die oben angegebene Bedeutung haben mit einer Grignardverbindung der Formel IV

R⁴-MgHal IV,

in welcher R⁴ Methyl, Vinyl, Allyl oder Benzyl bedeutet und in der Hal Chlor, Brom oder Jod bedeutet, erhalten. Bei dieser Umsetzung verfährt man so, daß man 0,8 bis 2,4 Äquivalente der Grignardverbindung einsetzt, vorzugsweise arbeitet man in Anwesenheit eines Lösungs- oder Verdünnungsmittels und gegebenenfalls in Anwesenheit eines ausbeutesteigernden Salzes.

Als Lösungsmittel kommen bevorzugt Ether in Frage, wie Diethylether, Di-n-propylether, Tetrahydrofurane oder Anisol, ferner tertiäre Amine wie N,N-Diethylanilin sowie Phosphorsäure-tris(dimethylamid); gegebenenfalls kann man die Reaktion auch im Gemisch dieser Lösungsmittel mit aliphatischen oder aromatischen Kohlenwasserstoffen wie n-Hexan oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide wie z.B. Tetra-n-butylammoniumchlorid in Frage.

Die Reaktionstemperaturen lassen sich je nach Lösungsmittel zwischen 0 und 100°C variieren, bevorzugt sind Temperaturen zwischen 0 und 60°C. Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extraktion, Umkristallisieren oder Chromatographie gereinigt.

Das Verfahren zur Herstellung der Ester der Formel I (R⁷ = C₂-C₄-Alkanoyl) besteht darin, daß man die sekundären oder tertiären Alkohole der Formel I (Z = CR⁴OR⁵ mit R⁵ = H) mit den entsprechenden Säureanhydriden in Gegenwart eines säurebindenden Mittels und gegebenenfalls in Gegenwart eines aprotischen Lösungsmittels oder Verdünnungsmittels sowie bevorzugt in Gegenwart eines Alkylierungskatalysators bei Temperaturen zwischen 0 und 100°C, vorzugsweise zwischen 10 und 50°C umsetzt. Als säurebindende Mittel können anorganische Basen wie Natriumamid oder besonders bevorzugt Pyridin in mindestens äquivalenten Mengen eingesetzt werden. Als Acylierungskatalysatoren verwendet man zweckmäßig Imidazol oder 4-Dimethylaminopyridin in Mengenanteilen von 0,01 bis 0,4 Äquivalenten, falls nicht bereits Pyridin anwesend ist. Als Lösungsmittel können Kohlenwasserstoffe, wie Cyclohexan oder Toluol, Ether wie Diethylether oder auch überschüssige säurebindende Amine wie Triethylamin oder Pyridin eingesetzt werden.

Die Überführung der Verbindungen der Formel III in die Verbindungen der Formel I erfolgt gemäß Anspruch 4 durch Halogenierung mit elementarem Fluor, Chlor oder Brom oder mit ähnlich reagierenden Halogenierungsmitteln gegebenenfalls in Gegenwart inerter Verdünnungsmittel. Derartige Reaktionen von Alkenen anderen Typs sind beschrieben, z.B. in den Methoden der Organischen Chemie (Houben-Weyl, Bd. V, 4. Ausgabe 1972, beispielsweise für Bromierungen, S. 39 ff).

Zur Bromierung kann beispielsweise in Methylenchlorid, Chloroform oder Tetrachlorkohlenstoff bei 0 bis 30°C gearbeitet werden. Höhere Temperaturen beschleunigen die Reaktionsgeschwindigkeit; bei tieferen Temperaturen läuft die Reaktion langsamer. Zur Umsetzung wird ein Äquivalent des Halogenierungsmittels z.b. elementares Brom im Falle der Bromierung verwendet.

Die Verbindungen der Formel III werden hergestellt, indem man bekannte Ketone (DE-26 38 470, 30 25 879, 32 20 183, 30 26 140) der Formel Azol-CH₂-CO-R² oder deren Alkalienolate mit Alkenylhalogeniden der Formel 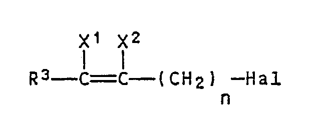 in Gegenwart einer Base alkyliert.

Falls gewünscht, können die neuen Verbindungen der Formel I auch in Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist.

Ferner lassen sich die Verbindungen der Formel I nach bekannten Methoden in Metallkomplexe überführen. Das kann durch Umsetzung dieser Verbindungen mit Metallsalzen, z.B. Salzen von Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die Herstellung der neuen Verbindungen der Formel I wird durch folgende Beispiele erläutert:

### Beispiel 1

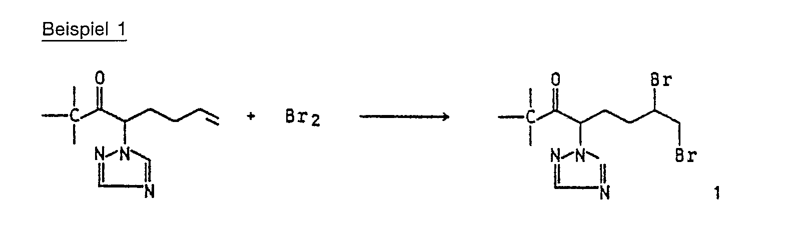

Zu 20 g (0,09 mol) 2,2-Dimethyl-4-(1,2,4-triazol-1-yl)-oct-7-en-3-on in 150 ml CCl₄ werden 4,5 ml (0,09 mol) Brom getropft. Eintropfendes Brom wird augenblicklich entfärbt. Nach beendeter Zugabe wird 2 × mit NaHCO₃ Lösung ausgeschüttelt, die CCl₄-Phase getrocknet (mit MgSO₄) und im Vakuum eingedampft: 26,4 g gelbliches Öl, 7,8-Dibrom-2,2-dimethyl-4-(1,2,4-triazol-1-yl)-octan-3-on (Verbindung Nr. 1).

Charakteristische Daten, ¹H-NMR in CDCl₃ (1,21+1,22) * ⁾s (9) 3,55m (1), 3,83m (1), (4,1+4,2) * ⁾m (1), (5,59+5,60) * ⁾t (1), (7,91+7,92)s (1), (8,39+8,40) * ⁾ ^{s (1)}
* ⁾Signalverdopplung, da Diastereomeres

### Beispiel 2

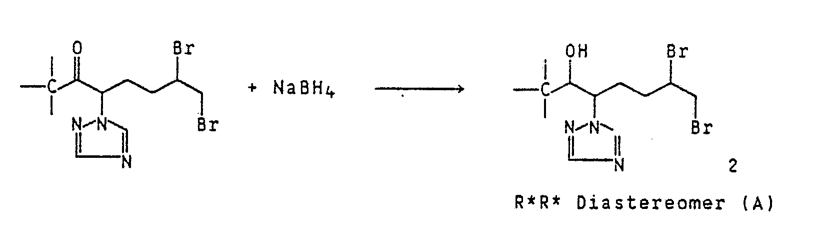

138,8 g (0,36 mol) 7,8-Dibrom-2,2-dimethyl-4-(1,2,4-triazol-1-yl)-octan-3-on (Verbindung Nr. 1) werden in 500ml Methanol bei 0°C portionsweise mit 13,8 g Natriumboranat versetzt. Nach beendeter Zugabe rührt man noch 3 Stunden bei Raumtemperatur. Man entfernt Methanol im Vakuum, verteilt zwischen Methylenchlorid und Wasser und wäscht die wäßrige Phase mit verdünnter Salzsäure neutral. Die Methylenchlorid-Phase wird mit Na₂SO₄ getroc knet. Nach Eindampfen im Vakuum wird mit Diisopropylether zur Kristallisation gebracht, abgesaugt und getrocknet. Es verbleiben 95 g 7,8-Dibrom-2,2-dimethyl-4-(1,2,4-triazol-1-yl)-octan-3-ol (R*R*Diastereomer (Verbindung Nr. 2), Fp.: 139°C.

### Beispiel3

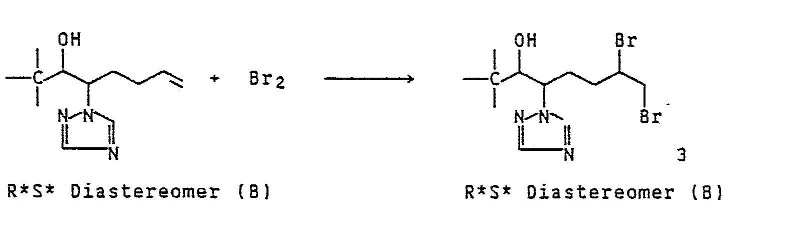

Zu 10 g (0,045 mol) 2,2-Dimethyl-4-(1,2,4-triazol-1-yl)-oct-7-en-3-ol in 100 ml CCl₄ werden bei Raumtemperatur 2,3 ml (0,045 mol) Brom zugetropft. Zur Aufarbeitung wird mit NaHCO₃-Lösung ausgeschüttelt, die CCl₄-Phase getrocknet (MgSO₄) und eingedampft. Anreiben mit Diisopropylether liefert 10,8 g 7,8-Dibrom-2,2-dimethyl-4-(1,2,4-triazol-1-yl)-octan-3-ol, R*S*-Diastereomer (Verbindung Nr. 3), Fp.: 119°C.

Weitere Beispiele sind in nachfolgender Tabelle zusammengestellt.

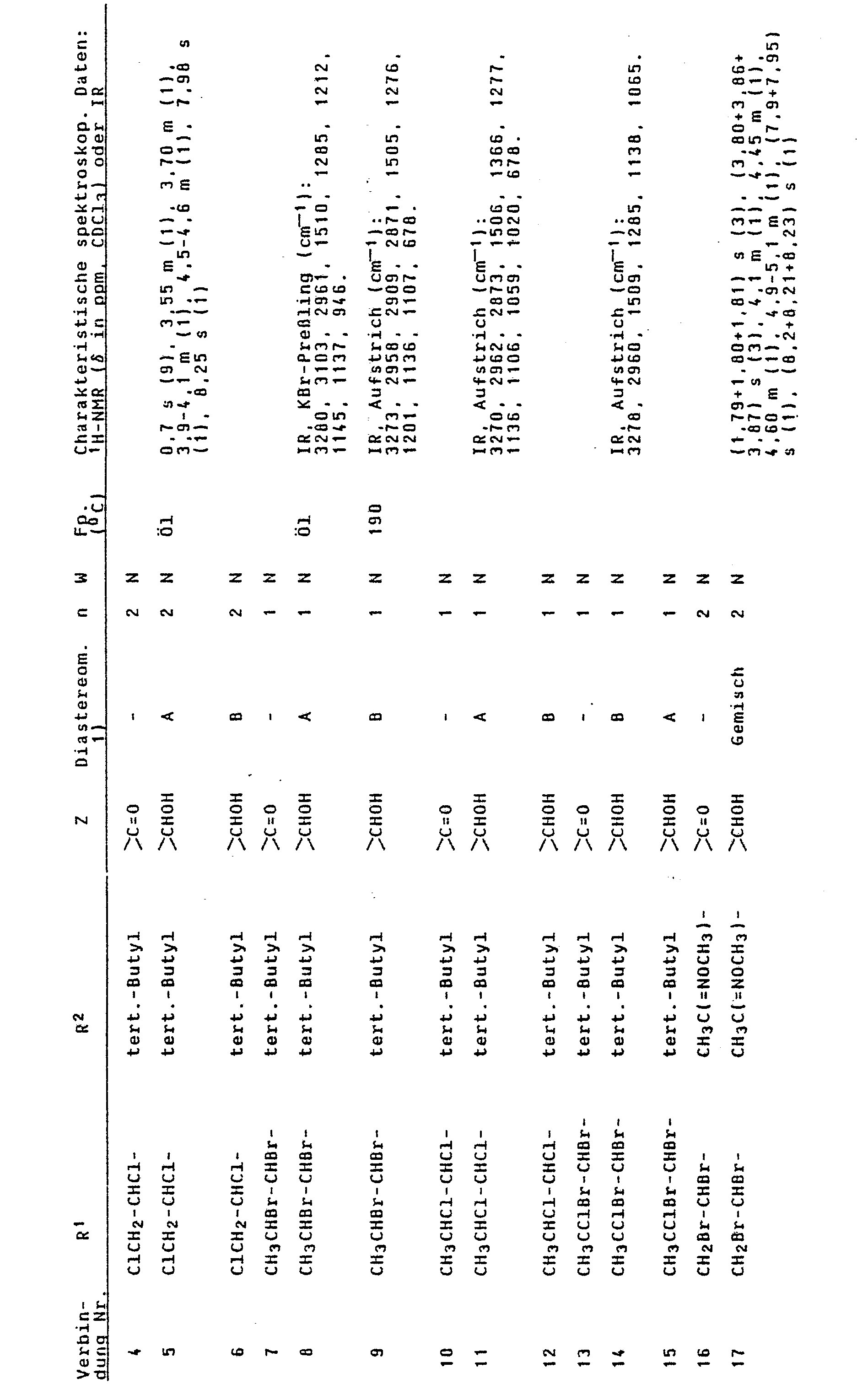

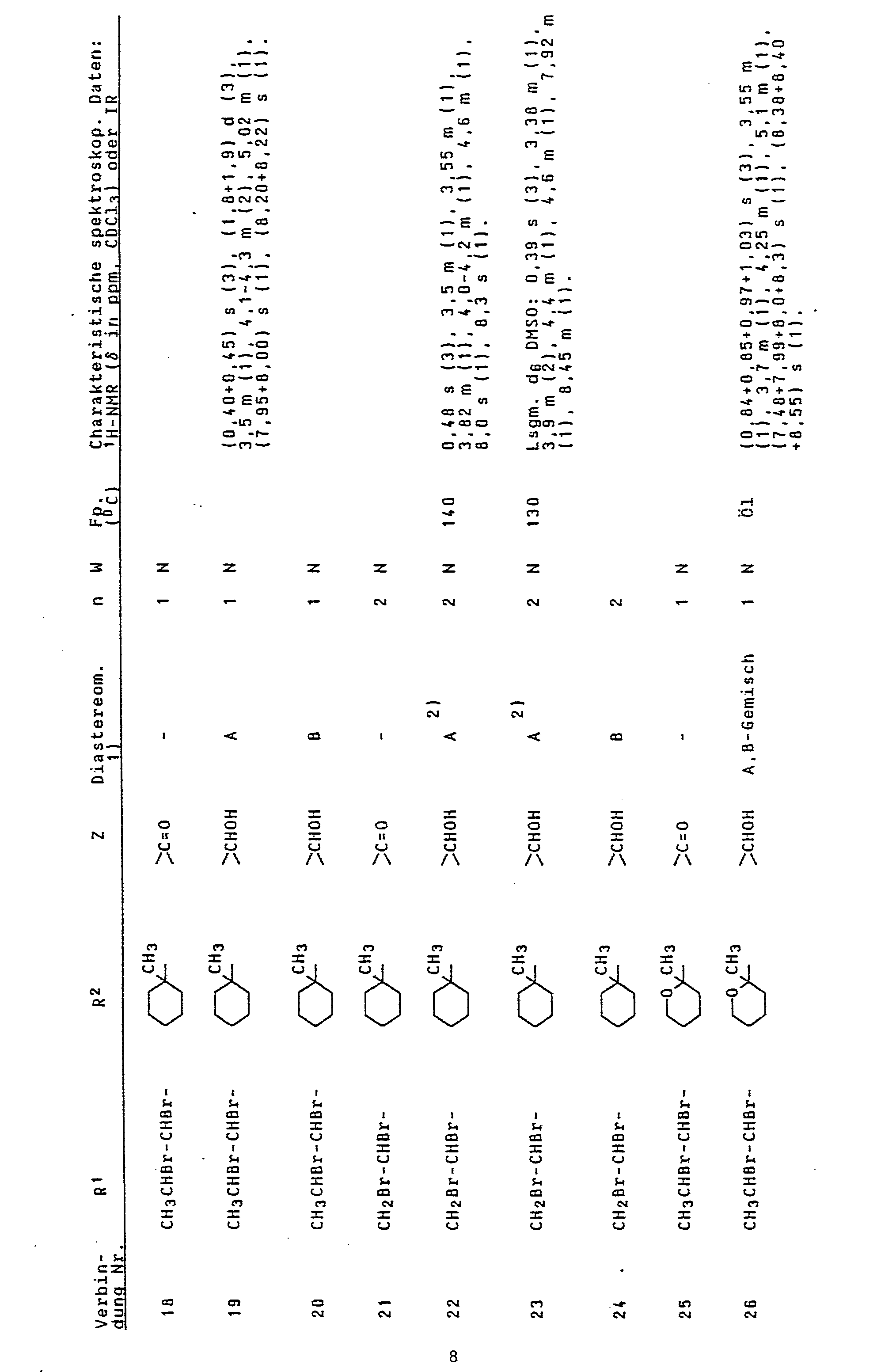

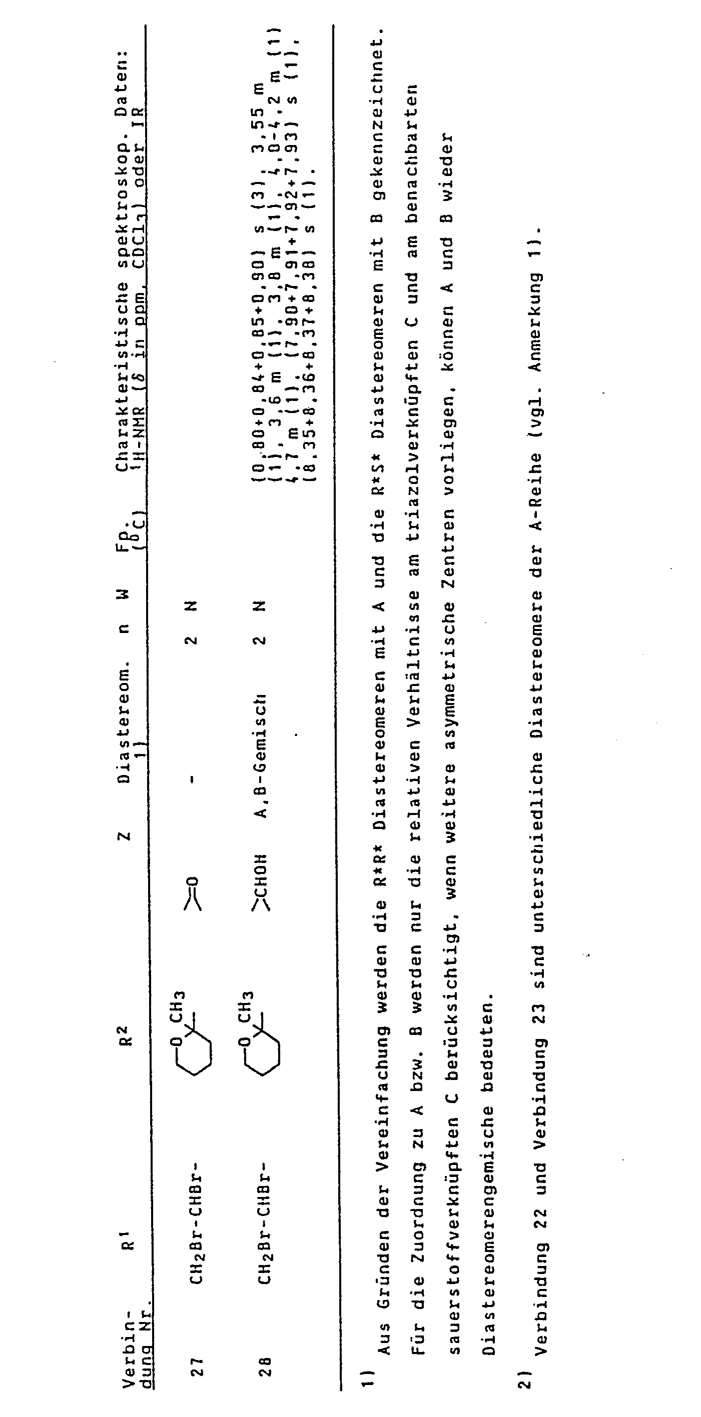

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyrenophora teres an Gerste,
Pyricularia oryzae an Reis,
Hemileia vastatrix an Kaffee,
Alternaria solani an Kartoffeln, Tomaten,
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalko hol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff. Die Aufwandmengen liegen je nach Art des gewünschten Effektes zwischen 0,02 und 3 kg Wirkstoff oder mehr je ha.

Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile lösemittelhaltiger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht. Einige der Verbindungen eignen sich auch zur Bekämpfung hautpathogener Pilze, z.B. Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:
I. Man vermischt 90 Gew.-Teile der Verbindung Nr. 3 mit 10 Gew.-Teilen N-Methyl-α-pyrrolidon und erhält eine Lösung, die zur Anwendung in Form kleinster Tropfen geeignet ist.
II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanol amid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.
III. 20 Gew.-Teile der Verbindung Nr.5 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IV. 20 Gew.-Teile der Verbindung Nr. 8 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
V. 80 Gew.-Teile der Verbindung Nr. 9 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.
VI. 3 Gew.-Teile der Verbindung Nr. 11 werden mit 97 Gew.-Teilen feinteiligem Kaolin innig vermischt. Man erhält auf diese Weise ein Stäubemittel, das 3 Gew.% des Wirkstoffs enthält.
VII. 30 Gew.-Teile der Verbindung Nr. 22 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 40 Gew.-Teile der Verbindung Nr. 19 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.
IX. 20 Gew.-Teile der Verbindung Nr. 23 werden mit 2 Gew.-Teilen Calciumsalz der Dodecylbenzolsulfonsäure, 8 Gew.-Teilen F ettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdith iocarbamat,
Mangan-Zink-ethylendiamin-bis-dithiocarbamat,
Tetra ethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propyle -bis-dithiocarbamat),
N,N′-Polypropylen-bis-(th iocarbamoyl)-disulfid;

Nitroderivate, wie
Dinitro-(1-methylheptyl)-phenylcrotonat,
2-sec-Butyl-4,6-di itrophenyl-3,3-dimethylacrylat,
2-sec-Butyl-4,6-din itrophenyl-isopropylcarbonat;
5-Nitro-isophthalsäure-di-isopropyles er

heterocyclische Substanzen, wie
2-Heptadecyl-2-imidazolin-acetat,
2,4-Dichlor-6-(o-chlorani ino)-s-triazin,
O,O-Diethyl-phthalimidophosphonothi oat,
5-Amino-1-[bis-(dimethylamino)-phosphvinyl]-3-phenyl-1,2,4-triazo ,
2,3-Dicyano-1,4-dithioanthrachinon,
2-Thio-1, 3-dithio-(4,5-b)-chinoxalin,
1-(Butylcarbamoyl)-2-benzimidazol-carb minsäuremethylester,
2-Methoxycarbonylamino-benzimi dazol,
2-(Furyl-(2))-benzimidazol,
2-(Thiazolyl-(4))-benzimidaz l,
N-(1,1,2,2-Tetrachlorethylthio)-tetrahydrophthal imid,
N-Trichlormethylthio-tetrahydrophthalimid,
N-Trichlormeth lthio-phthalimid,

N-Dichlorfluormethylthio-N ′,N′-dimethyl-N-phenyl-schwefelsäurediamid,
5-Ethoxy-3-tric lormethyl-1,2,3-thiadiazol,
2-Rhodanmethylthiobenzt hiazol,
1,4-Dichlor-2,5-dimethoxybenzol,
4-(2-Chlorphenylhydroa ano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
,3-Dihydro-5-carboxanilido-6-methyl-1,4-oxathiin-4,4-di oxid,
2-Methyl-5,6-dihydro-4H-pyran-3-carbonsäure-anilin,
2-Met yl-furan-3-carbonsäureanilid,
2,5-Dimethyl-furan-3- carbonsäureanilid,
2,4,5-Trimethyl-furan-3-carbonsäureanilid,
2 5-Dimethyl-furan-3-carbonsäurecyclohexylamid,
N-Cyc lohexyl-N-methoxy-2,5-dimethyl-furan-3-carbonsäureamid,
2-Methyl-be zoesäure-anilid,
2-Iod-benzoesäure-anilid,
N-Fo rmyl-N-morpholin-2,2,2-trichlorethylacetal,
Piperazin-1,4-diylbis-( -(2,2,2-trichlor-ethyl)-formamid,
1-(3,4-Dichlorani lino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylm rpholin,
N-[3-(p-tert.-Butylphenyl)-2-methylpropyl] -piperidin,
1-[2-(2,4-Dichlorphenyl)-4-ethyl-1,3-dioxolan-2-yl-ethy ]-1H-1,2,4-triazol
1-[2-(2,4-Dichlorphenyl)-4-n-pro pyl-1,3-dioxolan-2-yl-ethyl]-1H-1,2,4-triazol,
N-(n-Propyl)-N-(2,4, -trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol- 1-yl)-2-butanol,
α-(2-Chlorphenyl)-α -(4-chlorphenyl)-5-pyrimidin-methanol,
5-Butyl-2-dimethylamino-4-hydroxy-6-methyl-pyrimidin,
Bis-(p- hlorphenyl)-3-pyridinmethanol,
1,2-Bis-(3-ethoxycar bonyl-2-thioureido)-benzol,
1,2-Bis-(3-methoxycarbonyl-2-thioureido -benzol,

sowie verschiedene Fungizide, wie
Dodecylguanidinacetat,
3-[3-(3,5-Dimethyl-2-oxycyclohexyl)- -hydroxyethyl]-glutarimid,
Hexachlorbenzol,
DL- Methyl-N-(2,6-dimethyl-phenyl)-N-furoyl(2)-alaninat,
DL-N-(2,6-Dime hyl-phenyl)-N-(2′-methoxyacetyl)-alanin-methylester ,
N-(2,6-Dimethylphenyl)-N-chloracetyl-D,L-2-aminobutyrolacton,
-N-(2,6-Dimethylphenyl)-N-(phenylacetyl)-alaninmethyl ester,
5-Methyl-5-vinyl-3-(3,5-dichlorphenyl)-2,4-dioxo-1,3-oxazoli in,
3-[3,5-Dichlorphenyl(-5-methyl-5-methoxymethyl] -1,3-oxazolidin-2,4-dion,
3-(3,5-Dichlorphenyl)-1-isopropylcarbamoy hydantoin,
N-(3,5-Dichlorphenyl)-1,2-dimethylcyclop ropan-1,2-dicarbonsäureimid,
2-Cyano-[N-(ethylaminocarbonyl)-2-meth ximino]-acetamid,
1-[2-(2,4-Dichlorphenyl)-pentyl]- 1H-1,2,4-triazol,
2,4-Difluor-α-(1H-1,2,4-triazolyl-1-methyl)-benzh drylalkohol.

Für die folgenden Versuche wurde der bekannte Wirkstoff 2,2-Dimethyl-4-(1H-1,2,4-triazol-1-yl)-6-hepten-3-on (A) verwendet.

### Versuch 1

### Wirksamkeit gegen Weizenmehltau

Blätter von in Töpfen gewachsenen Weizenkeimlingen der Sorte Frühgold werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß bei der Anwendung als 0,025, 0,006 oder 0,0015%ige Spritzbrühe beispielsweise die Verbindungen 2, 3, 5, 8, 9 und 11 eine bessere fungizide Wirkung zeigen (100 %) als der bekannte Wirkstoff A (90 %).

### Versuch 2

### Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß bei der Anwendung als 0,025 %ige Spritzbrühe beispielsweise die Verbindungen 2, 3, 5, 8, 9, 11, 19, 22 oder 23 eine bessere fungizide Wirkung zeigen (97 %) als der bekannte Wirkstoff A (50 %).

### Versuch 3

### Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

Das Erge bnis des Versuches zeigt, daß bei der Anwendung als 0,025 %ige oder 0,006 %ige Spritzbrühe beispielsweise die Verbindungen 2, 3, 5, 8 und 11 eine bessere fungizide Wirksamkeit zeigen (97 %) als der bekannte Wirkstoff A (10 %).

### Versuch 4

### Wirksamkeit gegen Botrytis cinerea an Paprika

Paprikasämlinge der Sorte "Neusiedler Ideal Elite" werden, nachdem sich 4-5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß bei der Anwendung als 0,05 %ige Wirkstoffbrühe beispielsweise die Verbindungen 2, 3, 5, 8, 9, 11, 14, 19, 22 und 23 eine bessere fungizide Wirkung zeigen (97 %) als der bekannte Wirkstoff A (50 %).

### Versuch 5

### Wirksamkeit gegen Pyrenophora teres

Gerstenkeimlinge der Sorte "Asse" werden im Zweiblattstadium mit wäßrigen Suspensionen, die 80 % Wirkstoff und 20 % Emulgator in der Trockensubstanz enthalten, tropfnaß gespritzt. Nach 24 Stunden werden die Pflanzen mit einer Sporensuspension des Pilzes Pyrenophora teres inoculiert und für 48 Stunden in eine Klimakammer mit hoher Luftfeuchtigkeit bei 18°C gestellt. Anschließend werden die Pflanzen im Gewächshaus bei 20 bis 22°C und 70 % relativer Luftfeuchtigkeit für weitere 5 Tage kultiviert. Dann wird das Ausmaß der Symptomentwicklung ermittelt.

Das Ergebnis des Versuches zeigt, daß bei der Anwendung als 0,05 %ige Spritzbrühe beispielsweise die Verbindungen 2, 5, 8, 9, 11, 17, 19, 22 und 23 eine bessere fungizide Wirkung zeigen (97 %) als der bekannte Wirkstoff A (60 %).

Die Azolderivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation)
d) von den klimatischen Faktoren, z.B. Temperatur, Niederschlagsmenge sowie der Tageslänge und der Lichtintensität,
e) von der Bodenbeschaffenheit (einschließlich Düngung),
f) von der Formulierung bzw. Anwendungsform der Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.
   Als vorteilhaft für die Praxis erweist sich z.B. die Verringerung des Grasbewuchses an Straßenrändern, Decken, Kanalböschungen und auf Rasenflächen wie Park-, Sport- und Obstanlagen, Zierrasen und Flugplätzen, so daß der arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibaüme zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Blatt- und Sproßteil der Pflanze ist auch für ein gut kontrollierbares Entblättern von Nutzpflanzen wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künst lich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
   - die Öffnungsweite der Stromata reduziert wird
   - eine dickere Epidermis und Cuticula ausgebildet werden
   - die Durchwurzelung des Bodens verbessert wird
   - das Mikrok lima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha, bevorzugt 0,25 bis 3 kg/ha als ausreichend zu betrachten.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbeet gegossen.

Im Nachauflaufverfahren wurden die zu prüfenden Substanzen in wäßriger Aufbereitung auf die Pflanzen gesprüht oder auf das Substrat gegossen. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen:

### Vergleichssubstanz:

CCC = "A"

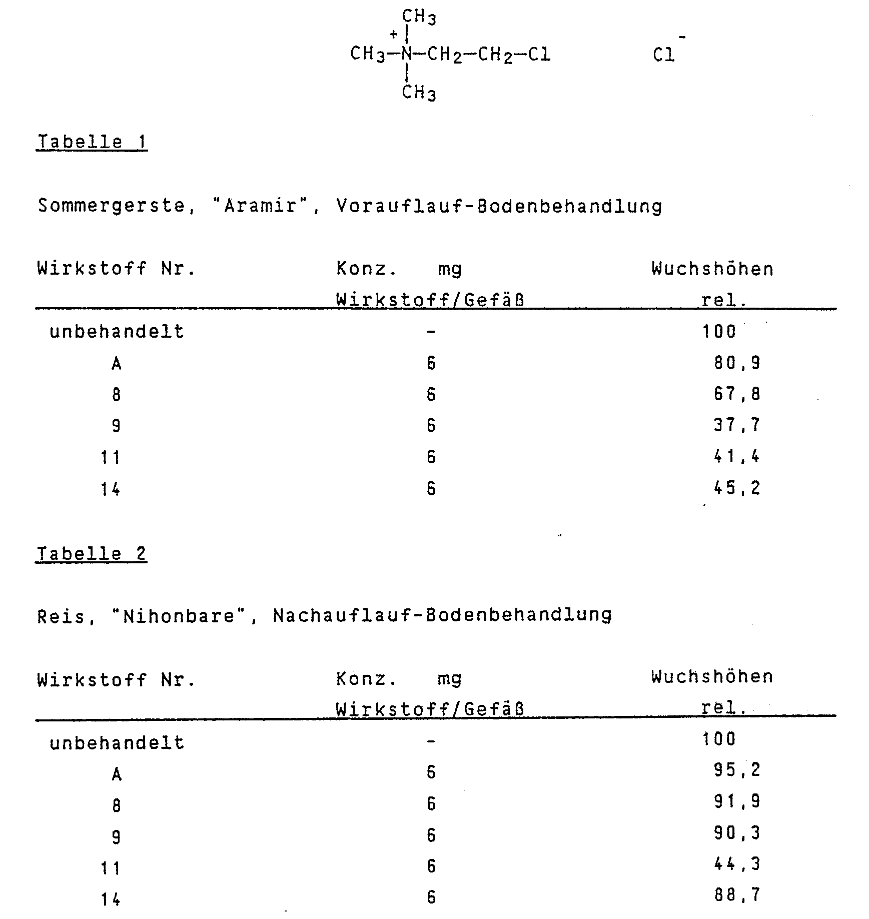

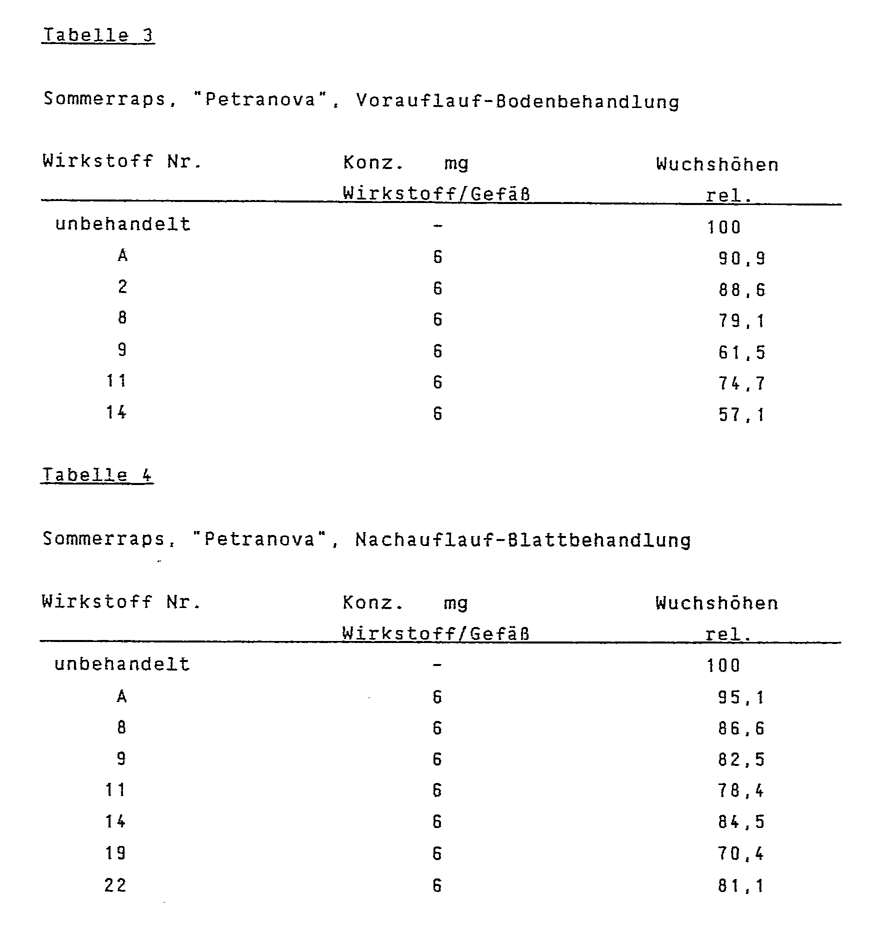

## Patentansprüche

1. Azolverbindungen der Formel I 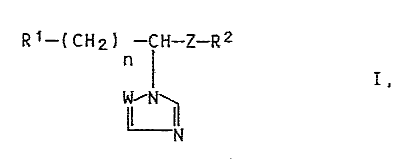 worin
R¹ für eine Gruppe der Formel 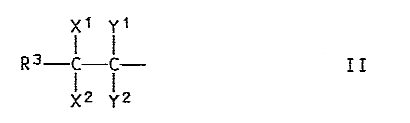 steht, wobei R³, X¹, X², Y¹ und Y² gleich oder verschieden sind und für Wasserstoff, Fluor, Chlor, Brom, Methyl oder Ethyl stehen, mit der Maßgabe, daß immer mindestens ein X und mindestens ein Y Fluor, Chlor oder Brom bedeutet,
n eine ganze Zahl von 1 bis 3 bedeutet, W ≧CH oder ≧N ist,
R² tert.-Butyl, 5-Methyl-1,3-dioxan-5-yl, ggf. durch Methyl substituiertes 1-Methylcyclohexyl oder ggf. durch Methyl substituiertes 2-Methyltetrahydropyran-2-yl oder die Gruppe 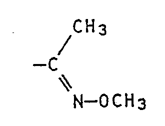 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
R⁵ Wasserstoff, C₂-C₄-Alkanoyl oder Benzoyl bedeutet,
sowie deren für Pflanzen verträgliche Säureadditionssalze und Metall-Komplexe.

2. Verfahren zur Herstellung von Azolverbindungen der Formel I gemäß Anspruch 1, worin Z die Gruppe =CR⁴-OR⁵ bedeutet und R⁴ Wasserstoff ist, dadurch gekennzeichnet, daß man ein Keton der Formel II 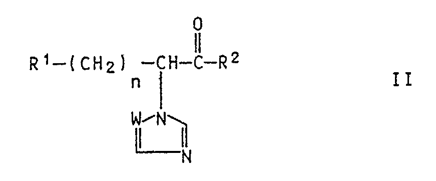 vorzugsweise stereoselektiv reduziert und den erhaltenen Alkohol gegebenenfalls mit einem Alkanoylhalogenid oder -anhydrid umsetzt.

3. Verfahren zur Herstellung von Azolverbindungen der Formel I gemäß Anspruch 1, worin Z die Gruppe =CR⁴-OR⁵ bedeutet und R⁴ Methyl, Vinyl, Allyl oder Benzyl ist, dadurch gekennzeichnet, daß man ein Keton der Formel II 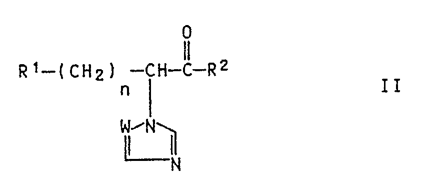 mit einer den Substituenten R⁴ einführenden Grignard- oder Lithiumverbindung umsetzt, das erhaltene Alkoholat hydrolysiert und den erhaltenen tertiären Alkoh nenfalls mit einem Alkanoylhalogenid oder -anhydrid umsetzt.

4. Verfahren zur Herstellung von Azolverbindungen der Formel I gemäß Anspruch 1, dadurch gekennzeichnet, daß man ein Alken der Formel III 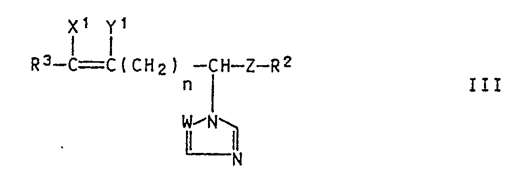 worin X, Y, n, R², R³, Z und W die in Anspruch 1 definierte Bedeutung haben, mit einem Fluorierungs-, Chlorierungs- oder Bromierungsmittel umsetzt.

5. Fungizides Mittel, enthaltend einen Trägerstoff und einen Wirkstoff der Formel 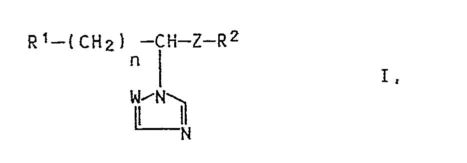 worin
R¹ für eine Gruppe der Formel 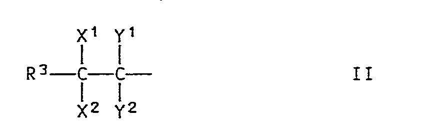 steht, wobei R³, X¹, X², Y¹ und Y² gleich oder verschieden sind und für Wasserstoff, Fluor, Chlor, Brom, Methyl oder Ethyl stehen, mit der Maßgabe, daß immer mindestens ein X und mindestens ein Y Fluor, Chlor oder Brom bedeutet,
n eine ganze Zahl von 1 bis 3 bedeutet,
W ≧CH oder ≧N ist,
R² tert.-Butyl, 5-Methyl-1,3-dioxan-5-yl, ggf. durch Methyl substituiertes 1-Methylcyclohexyl oder ggf. durch Methyl substituiertes 2-Methyltetrahydropyran-2-yl oder die Gruppe 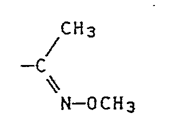 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
R⁵ Wasserstoff, C₂-C₄-Alkanoyl oder Benzoyl bedeutet,
sowie deren für Pflanzen verträgliche Säureadditionssalze und Metall-Komplexe.

6. Mittel zur Regulierung des Pflanzenwachstums, enthaltend einen Trägerstoff und einen Wirkstoff der Formel 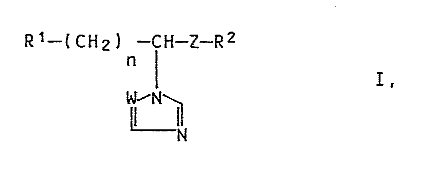 worin
R¹ für eine Gruppe der Formel 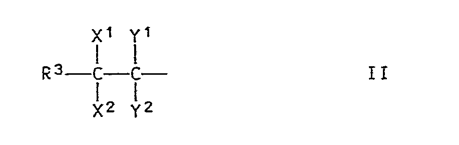 steht, wobei R³, X¹, X², Y¹ und Y² gleich oder verschieden sind und für Wasserstoff, Fluor, Chlor, Brom, Methyl oder Ethyl stehen, mit der Maßgabe, daß immer mindestens ein X und mindestens ein Y Fluor, Chlor oder Brom bedeutet,
n eine ganze Zahl von 1 bis 3 bedeutet,
W ≧CH oder ≧N ist,
R² tert.-Butyl, 5-Methyl-1,3-dioxan-5-yl, ggf. durch Methyl substituiertes 1-Methylcyclohexyl oder ggf. durch Methyl substituiertes 2-Methyltetrahydropyran-2-yl oder die Gruppe 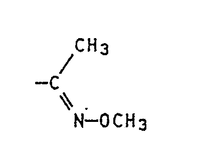 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
R⁵ Wasserstoff, C₂-C₄-Alkanoyl oder Benzoyl bedeutet,
sowie deren für Pflanzen verträgliche Säureadditionssalze und Metall-Komplexe.

7. Vefahren zur Bekämpfung von Pilzen, dadurch gekennzeichnet, daß man die Pilze oder die von Pilzbefall bedrohten Pflanzen, Materialien, Saatgüter oder den Boden mit einer fungizid wirksamen Menge einer Verbindung der Formel I 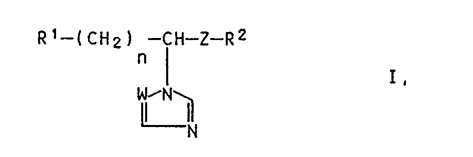 worin
R¹ für eine Gruppe der Formel 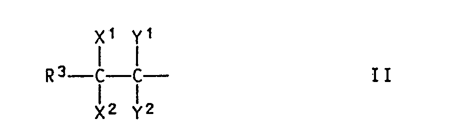 steht, wobei R³, X¹, X², Y¹ und Y² gleich oder verschieden sind und für Wasserstoff, Fluor, Chlor, Brom, Methyl oder Ethyl stehen, mit der Maßgabe, daß immer mindestens ein X und mindestens ein Y Fluor, Chlor oder Brom bedeutet,
n eine ganze Zahl von 1 bis 3 bedeutet,
W ≧CH oder ≧N ist,
R² tert.-Butyl, 5-Methyl-1,3-dioxan-5-yl, ggf. durch Methyl substituiertes 1-Methylcyclohexyl oder ggf. durch Methyl substituiertes 2-Methyltetrahydropyran-2-yl oder die Gruppe 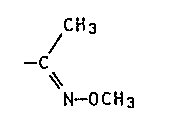 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein erstoff, C₂-C₄-Alkanoyl oder Benzoyl bedeutet,
sowie deren für Pflanzen verträgliche Säureadditionssalze und Metall-Komplexe behandelt.

8. Verfahren zur Regulierung des Pflanzenwachstums, dadurch gekennzeichnet, daß man eine Verbindung der Formel I 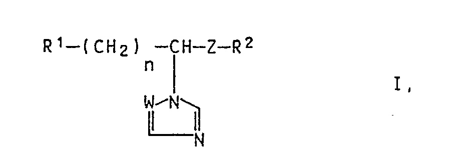 worin
R¹ für eine Gruppe der Formel 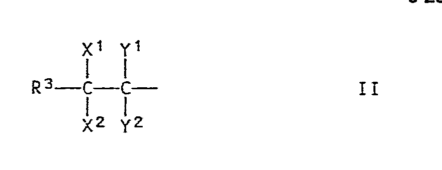 steht, wobei R³, X¹, X², Y¹ und Y² gleich oder verschieden sind und für Wasserstoff, Fluor, Chlor, Brom, Methyl oder Ethyl stehen, mit der Maßgabe, daß immer mindestens ein X und mindestens ein Y Fluor, Chlor oder Brom bedeutet,
n ein ganze Zahl von 1 bis 3 bedeutet,
W ≧CH oder ≧N ist,
R² tert.-Butyl, 5-Methyl-1,3-dioxan-5-yl, ggf. durch Methyl substituiertes 1-Methylcyclohexyl oder ggf. durch Methyl substituiertes 2-Methyltetrahydropyran-2-yl oder die Gruppe 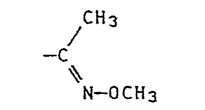 bedeutet, und
Z die Gruppe >C=O, >C=N-OH oder >CR⁴-OR⁵ bedeutet, und
R⁴ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
R⁵ Wasserstoff, C₂-C₄-Alkanoyl oder Benzoyl bedeutet,
sowie deren für Pflanzen verträgliche Säureadditionssalze und Metall-Komplexe auf Pflanzen, deren Lebensraum oder auf Saatgüter einwirken läßt.

9. Azolverbindung gemäß Anspruch 1, dadurch gekennzeichnet, daß
R¹ Dibromethyl, Dichlorethyl, Dibrompropyl, Dichlorpropyl,
n die Zahlen 1 oder 2
W Stickstoff
R² tert.-Butyl oder 1-Methylcyclohexyl und
Z die Gruppe CHOH
bedeutet.
